Europäisches Patentamt

European Patent Office

Office européen des brevets

(19)

(11) Publication number: **0 443 686 B1**

# EUROPEAN PATENT SPECIFICATION

(45) Date of publication of patent specification: **07.12.94**  (51) Int. Cl.⁵: **C07C 2/34**

(21) Application number: **91200367.0**

(22) Date of filing: **20.02.91**

The file contains technical information submitted
after the application was filed and not included in
this specification

(54) **Co-oligomerization process.**

(30) Priority: **22.02.90 GB 9004014**

(43) Date of publication of application:
**28.08.91 Bulletin 91/35**

(45) Publication of the grant of the patent:
**07.12.94 Bulletin 94/49**

(84) Designated Contracting States:
**BE DE ES FR GB IT NL**

(56) References cited:
**EP-A- 0 257 696**
**EP-A- 0 277 003**
**EP-A- 0 366 212**

**JOURNAL OF THE AMERICAN CHEMICAL SO-
CIETY, vol. 111, no. 7, 29th March 1989, pages
2728-2729, American Chemical Society; G.G.
HLATKY et al.: "Ionic, base-free zirconocene
catalysts for ethylene polymerization"**

(73) Proprietor: **SHELL INTERNATIONALE RE-
SEARCH MAATSCHAPPIJ B.V.
Carel van Bylandtlaan 30
NL-2596 HR Den Haag (NL)**

(72) Inventor: **Doyle, Michael John
Badhuisweg 3
1031 CM Amsterdam (NL)**
Inventor: **Terlouw, Willem
Badhuisweg 3
1031 CM Amsterdam (NL)**

**Description**

This invention relates to a co-oligomerization process and is particularly directed to the co-oligomerization of ethene and an alpha olefin.

Oligomerization processes for the production of linear olefins are well known. Thus for example $C_6$-$C_{20}$ linear olefins may be prepared from lower olefins such as ethene by oligomerization in the presence of a Ziegler catalyst system. Dependent on the conditions of temperature and pressure and the catalyst employed, some variation is possible in the resulting product composition. While linear olefins, especially linear alpha olefins, over a range of carbon chain lengths have found use as valuable intermediates in the preparation of polyolefins, detergents and lubricant additives, there is an increasing desire to shift the product slate towards the shorter chain length oligomers, eg $C_6$-$C_{10}$, which are more suited as intermediates in the preparation of linear low density polyethylene. However, the consequence of such a shift in product slate towards shorter chain length oligomers is a rise in the production of the less valuable ethene oligomerization product, 1-butene. There is therefore a need to provide a process for the conversion of 1-butene, and other lower alpha olefins, to useful linear olefin oligomers of increased chain length.

The applicants have found that lower linear alpha olefins such as propene, 1-butene and 1-pentene can be co-oligomerized with ethene to yield oligomeric products of high linearity and with a high alpha olefinic content in the presence of a catalyst system comprising a Group IVA organometallic compound and a boron compound such as a carborane. This is highly surprising, as such catalyst systems have previously been proposed as olefin polymerization catalysts, see for example, EP-A-277003, where the use of such a catalyst to copolymerize ethene and 1-butene resulted in a highly branched polymeric product. Other methods for the co-dimerization and co-oligomerization, of ethene and linear alpha olefins are known, but formation of higher linear alpha olefins is small and those linear olefins which are formed are predominantly internal olefins - see for example, "Comprehensive Organometallic Chemistry", 1982, published by Pergamon Press, Vol. 8, Section 52, and J. Pol. Sci. Part A: Polymer Chem., Vol. 27, 1989, pages 605-637.

According to the present invention, there is provided a process for the preparation of linear olefins comprising reacting ethene with at least one further alpha olefin under oligomerizing conditions, the reaction temperature being in the range of from 90 to 125 °C, in the presence of a catalyst system comprising a combination of a first component which is a bis(cyclopentadienyl) Group IVA metal compound containing a substituent capable of reacting with a cation and a second component which is a compound having a bulky anion containing a plurality of boron atoms and which is substantially non-coordinating under the reaction conditions and a cation and recovering an oligomeric product comprising linear olefins.

Metals of Group IVA are as defined in the Periodic Table of the Elements published in Kirk-Othmer, Encyclopaedia of Chemical Technology, 2nd edition, Vol. 8, p. 94.

The starting reactants comprise ethene, which may be supplied in the form of an ethene-containing gas together with an inert diluent such as nitrogen or helium. The further alpha olefin is preferably a lower linear alpha olefin such as propene, 1-butene or 1-pentene. However it is also possible to employ branched alpha olefin co-monomers such as 4-methyl-1-pentene, or an aryl-substituted olefin such as styrene or allyl benzene, dependent on the desired oligomerization products. Mixtures of alpha olefins may be employed. A preferred alpha olefin co-monomer is 1-butene. A particularly suitable source of co-monomer is the $C_4$ olefin fraction derived from an ethene oligomerization process to produce alpha olefins having from 4 to 20 carbon atoms. Such processes are known from, for example, EP-A-295960, US 4486615 and EP-A-241596.

To effect oligomerization, the reaction is suitably carried out at elevated temperatures, in the range of from 90 to 125 °C. The reaction is suitably carried out under conditions of moderate elevated pressure, preferably in the range of from 1 to 100 bar, more preferably from 5 to 60 bar. The optimum conditions of temperature and pressure used for a particular catalyst system to maximize the yield of oligomer and minimize competing reactions such as dimerization and polymerization can readily be established by the man skilled in the art. The conditions of temperature and pressure are preferably selected to yield a product slate with a "K factor" within the range of from 0.3 to 0.8. The K factor, which is indicative of the relative proportions of the product olefins, is the molar ratio of $[C_{n+2}]/[C_n]$ calculated from the slope of the graph of log $[c_n mol\%]$ versus n, where n is the number of carbon atoms in a particular product olefin.

The relative proportions of starting monomers can vary over a wide range. Suitably the amount of ethene to alpha olefin co-monomer lies within the range of 0.01 to 100 moles of ethene per mole of alpha olefin co-monomer, preferably 0.1 to 10 moles of ethene per mole of alpha olefin co-monomer.

The catalyst system, which may be formed initially prior to introduction to the reaction vessel, or which may be formed in situ, comprises a combination of a first component, which is a bis(cyclopentadienyl) Group IVA metal compound having a substituent capable of reacting with a proton and a second component which is an ionic combination of a bulky anion containing a plurality of boron atoms and a proton-donating cation, the anion being such that it is substantially non-coordinating under the reaction conditions employed. Thus, it is intended that the anion should not coordinate, or at least coordinate only weakly, to the bis-(cyclopentadienyl) metal entity which is formed by reaction of the donated proton and the acceptor substituent of the first compound. Examples of such catalyst systems, normally regarded as polymerization catalysts, are to be found in EP-A-277003 and the paper by Hlatky et al, J. Am. chem. Soc., 1989, Vol. 111 p. 2728-2729.

The first component is preferably a compound of zirconium or hafnium. The compound preferably has the formula $(Cp)_2 MR_1 R_2$ where each group Cp, which may be the same or different, represents a substituted or unsubstituted cyclopentadienyl group, M represents a Group IVA metal atom, preferably zirconium or hafnium, and $R_1$ and $R_2$ which may be the same or different, each represent a hydrogen atom or a substituted or unsubstituted hydrocarbyl group. Preferably each group cp represents an unsubstituted cyclopentadienyl group or a pentamethylcyclopentadienyl group. $R_1$ and $R_2$ are preferably alkyl groups such as methyl.

Such complexes are known and can be prepared for example by the routes described in "Chemistry of Organo-Zirconium and Hafnium Compounds", by Lappert et al., published by John Wiley & Sons.

The second component preferably contains, as the boron containing substantially non-coordinating anion, a carborane anion, suitably a carborane anion of formula $B_{11} CH_{12}{}^-$, while the cation is preferably a proton donating cation, preferably a quaternary ammonium cation such as tributyl ammonium. Alternatively the cation may be a metal cation, such as a silver ion. Such carboranes are known and can be prepared for example by methods such as that of Shelly et al, JACS, 1985, Vol. 107, p. 5955 to 5959. Other bulky boron containing anions may be used such as a tetra (perfluorophenyl) boron anion.

The catalyst system may be formed by mixing together the two components, preferably in solution in a solvent such as toluene to form a homogeneous catalyst system. The two compounds are generally employed in substantially equimolar amounts. However the mole ratio of the first compound to the second compound may vary within the range of from 0.1 to 5.0.

The oligomerization is generally, although not necessarily, carried out in an inert liquid solvent which is suitably also the solvent for the catalyst components. The reaction can be carried out in batch or continuous operation. Reaction times of from 1 minute to 5 hours have been found to be suitable, dependent on the activity of the catalyst. After a suitable reaction time, a conventional catalyst deactivating agent such as methanol, or other alcohol, may be added if desired to the reaction mixture to terminate the reaction. The resulting mixed olefins preferably have a chain length of from 5 to 20 carbon atoms. The reaction is preferably carried out in the absence of air or moisture.

Product olefins are recovered suitably by distillation and further separated as desired by distillation techniques dependent on the intended end use of the olefins.

The invention will now be further described with reference to the following examples.

Examples 1 to 9

Catalyst liquors A and B were prepared having the following compositions:
Catalyst liquor A.
bis(cyclopentadienyl) zirconium dimethyl (0.251g; 1.00 mmol)
tri-n-butyl ammonium 1-carbadodecacarborate of formula $Bu_3 NHB_{11} CH_{12}$ (0.329 g; 1.00 mmol)
toluene 300ml.
Catalyst liquor B.
bis(pentamethylcyclopentadienyl) zirconium dimethyl (0.391 g; 1.00 mmol)
$Bu_3 NHB_{11} CH_{12}$ (0.329 g ; 1.00 mmol)
toluene 300 ml.
Catalyst liquor C.
Bis(cyclopentadienyl) hafnium dichloride (1.0mmol) was mixed with methyl lithium (2.0mmol) in diethylether to give bis(cyclopentadienyl) hafnium dimethyl in situ. The ether was removed and toluene (300ml) and tri-n-butyl ammonium 1-carbadodecacarborate (1.0mmol) added to give catalyst liquor C.

Catalyst liquors A, B and C were employed in the co-oligomerization of ethene with 1-pentene (Examples 1 to 3 and 8), 1-butene (Example 4) and propene (Examples 5 and 6) under conditions of temperature, pressure and time given in Table 1 below. Examples 7 and 9 are comparative examples relating to ethene oligomerization with no added co-monomer. In each case the catalyst liquor was added to an autoclave (500 ml volume) containing the co-monomer together with toluene (300 ml), the reactor was then pressurized with ethene and rapidly heated to the reaction temperature. Pressure was maintained by continuous recharging of consumed ethene. At the end of the desired reaction time the reaction was terminated by treatment with methanol or exposure to air. The product distribution was determined by gas chromatography and the K factor, as previously defined, calculated by linear regression for each catalyst both for the even carbon number olefins ("e-olefins") and the odd carbon number olefins ("o-olefins"). The results are given in Table 1. It is to be noted that the distribution figures include the residual alpha olefin co-monomer.

Table 1

| Example No. | 1 | 2 | 3 | 4 | 5 | 6 | 7 | 8 | 9 |
|---|---|---|---|---|---|---|---|---|---|
| Catalyst | A | A | B | A | A | A | A | C | C |
| Co-monomer | $1\text{-}C_5^=$ | $1\text{-}C_5^=$ | $1\text{-}C_5^=$ | $1\text{-}C_4^=$ | $C_3^=$ | $C_3^=$ | – | $1\text{-}C_5^=$ | – |
| Amount (g or bar $C_3^=$) | 6.4 | 12.8 | 12.8 | 20.7 | 8 bar | 5 bar | – | 6.4 | – |
| Temperature (± 5°C) | 90 | 125 | 125 | 90 | 90 | 125 | 90 | 90 | 90 |
| Pressure (± 2 bar) | 10 | 20 | 20 | 20 | 10 | 30 | 20 | 20 | 20 |
| Time (minutes) | 30 | 30 | 1 | 30 | 30 | 30 | 30 | 30 | 30 |
| Product Distribution (g) | | | | | | | | | |
| $C_4^=$ | 4.44 | 6.50 | 1.43 | 15.76 | 1.03 | 7.02 | 5.61 | 0.27 | 2.73 |
| $C_5^=$ | 4.18 | 8.74 | 6.34 | – | 1.60 | 1.42 | – | 6.77 | – |
| $C_6^=$ | 4.29 | 6.38 | 2.16 | 7.28 | 1.74 | 6.49 | 6.05 | 0.33 | 5.61 |
| $C_7^=$ | 0.83 | 1.78 | 1.61 | – | 0.62 | 0.87 | – | 0.11 | – |
| $C_8^=$ | 3.06 | 4.43 | 2.07 | 6.16 | 0.42 | 4.85 | 5.21 | 0.36 | 5.10 |
| $C_9^=$ | 0.50 | 1.04 | 1.41 | – | 0.24 | 0.71 | – | 0.09 | – |
| $C_{10}^=$ | 2.00 | 2.96 | 1.87 | 5.06 | 0.17 | 3.14 | 4.40 | 0.33 | 4.05 |
| $C_{11}^=$ | 0.31 | 0.70 | 1.27 | – | 0.10 | 0.51 | – | 0.09 | – |
| $C_{12}^=$ | 1.47 | 2.14 | 1.73 | 4.50 | 0.08 | 2.48 | 3.98 | 0.31 | 3.35 |

Table 1 (continued)

| Example No. | 1 | 2 | 3 | 4 | 5 | 6 | 7 | 8 | 9 |
|---|---|---|---|---|---|---|---|---|---|
| $C_{13}=$ | 0.20 | 0.45 | 1.11 | – | 0.04 | 0.39 | – | 0.08 | – |
| $C_{14}=$ | 0.82 | 1.40 | 1.42 | 3.69 | 0.04 | 1.64 | 3.33 | 0.28 | 2.61 |
| $C_{15}=$ | 0.14 | 0.31 | 1.00 | – | 0.02 | 0.30 | – | 0.07 | – |
| $C_{16}$ | 0.54 | 0.87 | 1.23 | 3.19 | 0.03 | 1.17 | 2.87 | 0.25 | 2.00 |
| K factor e-olefins | 0.55 | 0.57 | 0.76 | 0.76 | 0.41 | 0.61 | 0.75 | 0.78 | 0.70 |
| o-olefins | 0.56 | 0.56 | 0.77 | – | 0.30 | 0.81 | – | 0.74 | – |
| Linearity (%w) e-olefins | 98.0 | 97.2 | 97.1 | 96.0 | 89.0 | 97.0 | 98.0 | 96.0 | 95.3 |
| o-olefins | 95.6 | 91.9 | 98.0 | – | 88.0 | 89.0 | – | 88.0 | – |
| Alpha olefins (%w) e-olefins | 37.5 | 62.9 | 86.0 | 47.0 | 35.2 | 67.0 | 45.9 | 76.0 | 74.0 |
| o-olefins | 40.0 | 59.6 | 85.0 | – | 34.8 | 63.0 | – | 75.0 | – |

## Claims

1. A process for the preparation of linear olefins comprising reacting ethene with at least one further alpha olefin under oligomerizing conditions, the reaction temperature being in the range of from 90 to 125°C, in the presence of a catalyst system comprising a combination of a first component which is a bis-

(cyclopentadienyl) Group IVA metal compound containing a substituent capable of reacting with a cation and a second component which is a compound having a bulky anion containing a plurality of boron atoms and which is substantially non-coordinating under the reaction conditions and a cation, and recovering an oligomeric product comprising linear olefins.

2. A process according to claim 1 wherein the further alpha olefin co-monomer comprises 1-butene.

3. A process according to claim 2 wherein the 1-butene is derived from a $C_4$ olefin fraction derived from an ethene oligomerization process.

4. A process according to any one of claims 1 to 4 wherein the oligomerization is carried out at a pressure within the range of from 1 to 100 bar.

5. A process according to any one of the preceding claims wherein the oligomerization conditions have been selected to yield an olefin product with a K factor within the range of from 0.3 to 0.8.

6. A process according to any one of the preceding claims wherein the ratio of ethene to alpha olefin co-monomer is within the range of 0.1 to 100 moles ethene per mole of co-monomer.

7. A process according to any one of the preceding claims wherein the first component of the catalyst system is a compound of formula $(Cp)_2 MR_1 R_2$ where each group Cp, which may be the same or different, represents a substituted or unsubstituted cyclopentadienyl group, M represents a group IVA metal atom, and $R_1$ and $R_2$, which may be the same or different, each represent a hydrogen atom or a substituted or unsubstituted hydrocarbyl group.

8. A process according to claim 8 wherein M is zirconium or hafnium.

9. A process according to claim 8 or 9 wherein each group Cp represents an unsubstituted cyclopentadienyl group or a pentamethylcyclopentadienyl group and $R_1$ and $R_2$ are each alkyl groups.

10. A process according to any one of the preceding claims wherein the second component of the catalyst system comprises a carborane anion as the substantially non-coordinating anion.

11. A process according to any one of the preceding claims wherein the second component of the catalyst system comprises a proton-donating cation.

12. A process according to claim 11 and 12 wherein the second component of the catalyst system is a trialkyl ammonium carborane, the carborane anion being represented by the formula $B_{11} CH_{12}-$.

**Patentansprüche**

1. Verfahren zur Herstellung von linearen Olefinen, umfassend das zur-Reaktion-bringen von Ethen mit mindestens einem weiteren alpha-Olefin unter oligomerisierenden Bedingungen, wobei die Reaktionstemperatur im Bereich von 90 bis 125°C liegt, in Gegenwart eines Katalysatorsystems, umfassend eine Kombination aus einer ersten Komponente, welche eine Bis(cyclopentadienyl)-Verbindung eines Metalles der Gruppe IVA ist, enthaltend einen Substituenten, der mit einem Kation reagieren kann, und einer zweiten Komponente, welche eine Verbindung mit einem voluminösen Anion, enthaltend eine Vielzahl von Boratomen und welches unter den Reaktionsbedingungen im wesentlichen nicht-koordinierend ist, und mit einem Kation ist, sowie das Gewinnen eines oligomerischen Erzeugnisses, umfassend lineare Olefine.

2. Verfahren nach Anspruch 1, in welchem das weitere alpha-Olefin-Co-Monomer 1-Buten umfaßt.

3. Verfahren nach Anspruch 2, in welchem das 1-Buten von einer $C_4$-Olefin-Fraktion abgeleitet ist, hergestellt durch ein Ethen-Oligomerisierungs-Verfahren.

4. Verfahren nach einem der Ansprüche 1 bis 3, in welchem die Oligomerisierung bei einem Druck im Bereich von 1 bis 100 bar durchgeführt wird.

**5.** Verfahren nach einem der vorhergehenden Ansprüche, in welchem die Oligomerisierungsbedingungen so ausgewählt worden sind, daß ein Olefinerzeugnis mit einem K-Faktor im Bereich von 0,3 bis 0,8 erhalten wird.

**6.** Verfahren nach einem der vorhergehenden Ansprüche, in welchem das Verhältnis von Ethen zum alpha-Olefin-Co-Monomer im Bereich von 0,1 bis 100 Mol Ethen pro Mol Co-Monomer liegt.

**7.** Verfahren nach einem der vorhergehenden Ansprüche, in welchem die erste Komponente des Katalysatorsystems eine Verbindung der Formel $(C_p)_2 MR_1 R_2$ ist, in welcher jede der Gruppen $C_p$, welche gleich oder unterschiedlich sein können, eine substituierte oder nicht substituierte Cyclopentadienylgruppe, M ein Metallatom der Gruppe IVA ist und $R_1$ und $R_2$, welche gleich oder unterschiedlich sein können, jeweils ein Wasserstoffatom oder eine substituierte oder nicht substituierte Kohlenwasserstoffgruppe darstellen.

**8.** Verfahren nach Anspruch 7, in welchem M Zirkon oder Hafnium ist.

**9.** Verfahren nach Anspruch 7 oder 8, in welchem jede der Gruppen $C_p$ eine nicht substituierte Cyclopentadienylgruppe oder eine Pentamethylcyclopentadienylgruppe ist und $R_1$ und $R_2$ jeweils Alkylgruppen sind.

**10.** Verfahren nach einem der vorhergehenden Ansprüche, in welchem die zweite Komponente des Katalysatorsystems ein Carborananion als das im wesentlichen nicht-koordinierende Anion umfaßt.

**11.** Verfahren nach einem der vorhergehenden Ansprüche, in welchem die zweite Komponente des Katalysatorsystems ein Proton abgebendes Kation umfaßt.

**12.** Verfahren nach den Ansprüchen 10 und 11, in welchem die zweite Komponente des Katalysatorsystems ein Trialkylammoniumcarboran ist, wobei das Carborananion durch die Formel $B_{11}CH_{12}$- dargestellt ist.

**Revendications**

**1.** Procédé pour la préparation d'oléfines linéaires, comprenant la réaction d'éthylène avec au moins une autre alpha oléfine dans des conditions d'oligomérisation, la température de réaction étant comprise dans l'intervalle de 90 à 125°C, en présence d'un système catalytique comprenant une combinaison d'un premier composant oui est un composé de bis(cyclopentadiényl) métal du groupe IVA contenant un substituant apte à réagir avec un cation et un second composant qui est un composé ayant un anion volumineux contenant une pluralité d'atomes de bore et qui est pratiquement non-coordinant dans les conditions de réaction et un cation, et la récupération d'un produit oligomère comprenant des oléfines linéaires.

**2.** Procédé selon le revendication 1, dans lequel l'autre co-monomère d'alpha oléfine comprend du 1-butène.

**3.** Procédé selon la revendication 2, dans lequel le 1-butène est dérivé d'une fraction oléfinique en $C_4$ dérivée d'un procédé d'oligomérisation d'éthylène.

**4.** Procédé selon l'une quelconque des revendications 1 à 3, dans lequel l'oligomérisation est effectuée sous une pression comprise dans l'intervalle de 1 à 100 bar.

**5.** Procédé selon l'une quelconque des revendications précédentes, dans lequel les conditions d'oligomérisation ont été choisies de façon à procurer un produit oléfinique ayant un facteur K compris dans l'intervalle de 0,3 à 0,8.

**6.** Procédé selon l'une quelconque des revendications précédentes, dans lequel le rapport de l'éthylène au co-monomère d'alpha oléfine est compris dans l'intervalle de 0,1 à 100 moles d'éthylène par mole de co-monomère.

7. Procédé selon l'une quelconque des revendications précédentes, dans lequel le premier composant du système catalytique est un composé de formule $(Cp)_2MR_1R_2$ où chaque groupe Cp, qui peut être le même ou différent, représente un groupe cyclopentadiényle substitué ou non-substitué, M représente un atome de métal du groupe IVA, et $R_1$ et $R_2$, qui peuvent être identiques ou différents, représentent chacun un atome d'hydrogène ou un groupe hydrocarbyle substitué ou non-substitué.

8. Procédé selon la revendication 7, dans lequel M est le zirconium ou le hafnium.

9. Procédé selon l'une des revendications 8 ou 9, dans lequel chaque groupe Cp représente un groupe cyclopentadiényle non-substitué ou un groupe pentaméthylcyclopentadiényle et $R_1$ et $R_2$ sont chacun des groupes alkyle.

10. Procédé selon l'une quelconque des revendications précédentes, dans lequel le second composent du système catalytique comprend un anion carborane en tant qu'anion pratiquement non-coordinant.

11. Procédé selon l'une quelconque des revendications précédentes, dans lequel le second composent du système catalytique comprend un cation donneur de protons.

12. Procédé selon l'une des revendications 10 et 11, dans lequel le second composent du système catalytique est un carborane de trialkyl ammonium, l'anion carborane étant représenté par la formule $B_{11}CH_{12}-$.